# EUROPEAN PATENT SPECIFICATION

(11) **EP 0 749 736 B1**
(45) Date of publication and mention of the grant of the patent: **26.01.2000**
(21) Application number: 95114597.8
(22) Date of filing: 16.09.1995
(51) Int. Cl.: A61F 13/15

(54) **Perforated dual topsheets for absorbent articles**
Zweifache perforierte Decklagen für absorbierende Artikel
Couche supérieure double avec perforations pour article absorbant

(30) Priority: 19.06.1995 EP 95830254
(43) Date of publication of application: 27.12.1996
(73) Proprietor: THE PROCTER & GAMBLE COMPANY, Cincinnati, Ohio 45202 (US)
(72) Inventor: Divo, Michael, D-61381 Friedrichsdorf (DE); Coles, Peter, I-66023 Francavilla al Mare, Chieti (IT); Fornasari, Giancarlo, I-Pescara (IT)
(74) Representative: Hirsch, Uwe Thomas

(56) References cited:
- EP-A- 0 165 807
- EP-A- 0 203 821
- EP-A- 0 205 286
- EP-A- 0 545 423
- US-A- 4 609 518

## Description

### Field of the invention

The present invention relates to absorbent articles of less than 8 mm thickness, particularly sanitary napkins having layered topsheets, see EP-A-0 545 423. In particular a first layer receiving the liquids to be absorbed comprises apertures of at least 1.4 mm² area in a film. These large apertures provide exceptionally good liquid intake performance for the absorbent article. A second film layer provides liquid transfere to an absorbent structure through apertures of less than 1.4 mm² area. Additionally the second layer provides visual masking of absorbent liquids in the area of the large apertures of the first topsheet layer. The absorbent structure comprises an absorbent structure which is free of absorbent hydrogel particles.

### Background of the invention

Sanitary articles such as sanitary napkins, baby diapers, absorbent inserts, and absorbent adult incontinence articles are well-known in the art. Typically all these articles comprise a wearer facing surface and a garment facing surface. The wearer facing surface receives from the wearer of such articles liquids, bodily discharges such as menses, to be absorbed. In order for the article to store the liquid the wearer facing surface has to be liquid permeable while maintaining integrity of the outer wearer facing surface of the absorbent article. This wearer facing surface is provided by a topsheet.

Well-known topsheets in the art of absorbent articles are non-woven fabrics, woven fabrics or films. Films have to be rendered permeable by aperturing. Fabrics or non-woven fabrics are made of fibers which by their nature provide non-linear apertures of varying and changing size depending on the selected direction for liquid transport through them. Films are often made of polymeric material and typically comprise apertures which have been engineered to provide certain characteristics. These apertures can vary in shape and size. The walls of the apertures define the amount of extension-if any- beyond the plane of the film thickness and the direction of such extensions. The film apertures also can be provided in the shape of a funnel.

A typical topsheet made of polyethylene film has been successfully used in sanitary articles and adult incontinence products as well as inserts and baby diapers. A dual layer film topsheet with identical layers has already been disclosed in US-A-4,323,069. However, this reference does not disclose apertures of the size required for the uppermost layer according to the present invention. One problem remaining for film topsheets in general is the total amount of liquid capable of passing through such a topsheet under usual usage conditions due to the total amount of open area of all apertures and individual aperture size and shape in particular. Exceptionally large apertures increase the liquid passage rate but pose the problem of masking because liquids such as menses remain visible to the wearer, which is considered undesirable. Also large apertures promote a backflow of absorbed liquid, so called rewet, which is undesirable. Small individual apertures on the other hand cannot provide the liquid passage characteristics required to let liquids of high surface tensions pass through; this can be a problem in an absolute sense for very small apertures or cause too low a rate of liquid flow.

It also has been found that the total amount of open area for a given aperture size and shape is approximately linearly related to the rate of liquid passage. Again, masking of the liquid which has passed through but also material strength and other appearance considerations are limiting the extend as to which the total open area in a film topsheet can be selected.

The problem of masking becomes even more acute if the absorbent structure underlying the topsheet does not store liquids in a solid or semi-solid form. In particular absorbent structures which do not comprise so called hydrogel particles are used when very soft or cushioning articles are desired since these hydrogel materials are known to increase stiffness or crush resistance for example form US 4,217,901. The drawback of only absorbing liquids in a fibrous or foam structure is that liquid can be released under pressure. The topsheet obviously needs to prevent a backflow of absorbent liquid but in particular topsheets with large apertures need to provide a high degree of masking since that communicates cleanliness and secure storage of liquids to the wearer.

Another requirement of absorbent articles having hydrogel particles is to prevent these particles migrating through the topsheet onto the user's skin. This becomes more difficult to satisfy as the apertures in the topsheet become larger. Obviously, however, the problem can be solved completely by eliminating the hydrogel particles. Certain users, those not having a particular high quantity of liquid to be stored but releasing the liquid in gustes, i.e. users with high acquisition but low/medium storage needs actually have been found to accept hydrogel particle free absorbent structures, under the provision that acquisition and rewet are not noticeably different from articles with hydrogel particles.

From the foresaid it is clear that a balancing problem between masking, material strength, other appearance considerations and total open area as well as individual aperture size and shape exists in the state of the art especially for articles having fibrous/foam absorbent structures in direct contact with the topsheet. The present invention does not attempt to provide selection criteria for this balancing problem but to shift the balance for this problem in order to obtain improved absorbent articles in respect to their capability of fast liquid intake as well as rewet and masking of the liquid received while maintaining acceptable characteristics of material strength and other considerations for topsheets.

It is hence an objective of the present invention to provide an apertured topsheet for articles which are hydrogel particle free, e.g. fibrous/foam absorbent articles, which topsheet has larger apertures than those commonly found acceptable in film apertured topsheets while effectively improving or at least not deteriorating the rewet and masking of absorbed liquids.

### Description of the invention

The present invention provides an absorbent article according to claim 1 having all the benefits of a large aperture film topsheet without the masking and rewet problems of the prior art. In particular, the absorbent article comprises a laminate topsheet having a wearer facing surface and a garment facing surface. The topsheet comprises a first and a second layer which are preferably joined to each other. An absorbent article generally further comprises a backsheet and an absorbent structure alternatively called absorbent core placed between the topsheet and the backsheet. The absorbent structure comprises no hydrogel particles. Absorbent articles according to the present invention also have a maximum thickness, typically in the center of the article, of 8 mm.

### Absorbent structure

The absorbent structure can include the following components: (a) optionally a primary fluid distribution layer preferably together with a secondary optional fluid distribution layer; (b) a fluid storage layer; (c) optionally a fibrous ("dusting") layer underlying the storage layer; and (d) other optional components. According to the present invention the absorbent structure does not comprise particulate hydrogel particles. The absorbent structure and its individual layers can typically be fibrous or made of foam material.

### a Primary/Secondary Fluid Distribution Layer

One optional component of the absorbent structure according to the present invention is a primary fluid distribution layer and a secondary fluid distribution layer. The primary distribution layer typically underlies the topsheet and is in fluid communication therewith. The topsheet transfers the acquired fluid to this primary distribution layer for ultimate distribution to the storage layer. This transfer of fluid through the primary distribution layer occurs not only in the thickness, but also along the length and width directions of the absorbent product. The also optional but preferred secondary distribution layer typically underlies the primary distribution layer and is in fluid communication therewith. The purpose of this secondary distribution layer is to readily acquire fluid from the primary distribution layer and transfer it rapidly to the underlying storage layer. This helps the fluid capacity of the underlying storage layer to be fully utilised. The fluid distribution layers can be comprised of any material typical for such distribution layers. In particular fibrous layers which maintain the capillaries between fibers even when wet are useful as distribution layers.

### b Fluid Storage Layer

Positioned in fluid communication with, and typically underlying the primary or secondary distribution layers, is a fluid storage layer. The fluid storage layer can comprise any usual absorbent material or combinations thereof. The fluid storage layer can comprise fibrous absorbent gelling materials in the form of fibers usually referred to as "hydrogel", "superabsorbent", hydrocolloid" fibers alone or in combination with suitable carriers such as other fibers or foams.

The absorbent gelling fibers are capable of absorbing large quantities of aqueous body fluids, and are further capable of retaining such absorbed fluids under moderate pressures. The absorbent gelling fibers can be dispersed homogeneously or non-homogeneously in a suitable carrier. The suitable carriers, provided they are absorbent as such, can also be used alone.

Suitable absorbent gelling materials for use herein will most often comprise a substantially water-insoluble, slightly cross-linked, partially neutralised, polymeric gelling material. This material forms a hydrogel upon contact with water. Such polymer materials can be prepared from polymerizable, unsaturated, acid-containing monomers which are well known in the art.

Suitable absorbent materials include materials which are conventionally utilised as carriers in absorbent structures which comprise hydrogel particles, including those materials useful to provide the optional primary or secondary fluid distribution layers. Again no hydrogel particles are present in the fluid storage layer according to the present invention. Such materials are natural, modified or synthetic fibers, particularly modified or non-modified cellulose fibers, in the form of fluff and/or tissues. Suitable absorbent materials can be used alone or in combinations. Most preferred are tissue or tissue laminates in the context of sanitary napkins/panty liners.

An embodiment of the absorbent structure made according to the present invention comprises a double layer tissue laminate formed by folding the tissue onto itself. These layers can be joined to each other for example by adhesive or by mechanical interlocking or by hydrogen bridge bonds. Other absorbent materials can be comprised between the layers.

Modified cellulose fibers such as the stiffened cellulose fibers can also be used. Synthetic fibers can also be used and include those made of cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibers, tricomponent fibers, mixtures thereof and the like. Preferably, the fiber surfaces are hydrophilic or are treated to be hydrophilic. The storage layer can also include filler materials, such as Perlite, diatomaceous earth, Vermiculite, etc., to improve liquid retention.

### c Optional Fibrous Layer

An optional component for inclusion in the absorbent structure according to the present invention is a fibrous layer adjacent to, and typically underlying the storage layer. This layer provides some additional fluid-handling capabilities such as rapid wicking of fluid along the length of the pad. This layer can be made from any of the above mentioned absorbent materials.

### d Other Optional Components of the absorbent structure

The absorbent structure according to the present invention can include other optional components normally present in absorbent webs. For example, a reinforcing scrim can be positioned within the respective layers, or between the respective layers, of the absorbent structure. Such reinforcing scrims should be of such configuration as to not form interfacial barriers to fluid transfer. Given the structural integrity that usually occurs as a result of thermal bonding, reinforcing scrims are usually not required for thermally bonded absorbent structures.

Another component which can be included in the absorbent structure according to the invention and preferably is provided close to or as part off the primary or secondary fluid distribution layer are odor control agents. Active carbon coated with or in addition to other odor control agents, in particular suitable zeolite or clay materials, are optionally incorporated in the absorbent structure. Also zeolite or clay materials alone can optionally be used as odour control materials. These components can be incorporated in any desired form but often are included as discrete particles immobilised in a laminate structure.

According to the present invention the thickness or caliper of the whole absorbent article is important. The major part of the thickness of an absorbent article results from the amount of absorbent material used in the absorbent structures. As is usual in the art the absorbent structure can be tailored to have more absorbent material deposited in the region which is intended to receive liquid to be absorbed. Such absorbent structures are called profiled. If profiled absorbent structures are employed the thickness of the absorbent article according to the present invention is taken at the thickest part of the absorbent structure.

### Backsheet

The backsheet primarily prevents the exudates absorbed and contained in the absorbent structure from wetting articles that contact the absorbent product such as underpants, pants, pyjamas and undergarments. The backsheet is preferably impervious to liquids (e.g. menses and/or urine) and is preferably manufactured from a thin plastic film, although other flexible liquid impervious materials can also be used. As used herein, the term "flexible" refers to materials that are compliant and will readily conform to the general shape and contours of the human body. The backsheet also can have elastic characteristics allowing it to stretch in one or two directions.

The backsheet typically extends across the whole of the absorbent structure and can extend into and form part of or all of the preferred sideflaps, side wrapping elements or wings as shown in Figure 3.

The backsheet can comprise a woven or nonwoven material, polymeric films such as thermoplastic films of polyethylene or polypropylene, or composite materials such as a film-coated nonwoven material. Preferably, the backsheet is a polyethylene film having a thickness of from about 0.012 mm (0.5 mil) to about 0.051 mm (2.0 mils).

Exemplary polyethylene films are manufactured by Clopay Corporation of Cincinnati, Ohio, under the designation P18-0401 and by Ethyl Corporation, Visqueen Division, of Terre Haute, Indiana, under the designation XP-39385. The backsheet is preferably embossed and/or matte finished to provide a more clothlike appearance. Further,. the backsheet can permit vapours to escape from the absorbent structure, i.e. be breathable, while still preventing exudates from passing through the backsheet. Also breathable backsheets comprising several layers, e.g. apertured or microporous film plus non-woven structures, can be used.

### The topsheet

The term "joined", a used herein, encompasses configurations in which the first layer is directly secured to the second layer by affixing the first layer directly to the second layer; configurations in which the first layer is indirectly secured to the second layer by affixing the first layer to intermediate layer(s) which in turn are affixed to the second layer. Both layers are preferably joined to each other across at least 25 % of their total surface.

The layers of the topsheet can be joined together by adhesives, stitching, heat and/or pressure bonds, dynamic mechanical bonds, ultrasonic bonds, intermingling or entanglement of structural elements comprising the layers of the topsheet, such as by extruding one layer onto another, or by any other means known in the art.

The topsheet comprises a first passage layer which provides the user facing surface of the topsheet and a second passage layer between the first passage layer and the absorbent structure.

The topsheet as a whole and hence each layer individually needs to be compliant, soft feeling, and non-irritating to the wearer's skin. It also can have elastic characteristics allowing it to be stretched in one or two directions.

The first passage layer is provided by a film material having apertures which are referred herein as "large apertures" and optionally apertures which are referred herein as "small apertures". These apertures are provided to facilitate liquid transport from the wearer facing surface towards the absorbent structure.

For all measurements regarding the apertures in the first passage layer the plane of the smallest cross sectional areas of the aperture should be used, unless otherwise mentioned.

The large apertures have an individual open area of from 1.4 mm² or more. Preferably the large apertures are in the range from 1.4 m² to 3.0 mm² and most preferably from 1.5 mm² to 2.5 mm². The total area of the large apertures in the upper passage layer excluding all other liquid transport apertures should be in the range from 5 % to 20 %, preferably from 10 % to 20 % of the surface area of the first layer of the topsheet.

The optional small apertures in the first layer of the topsheet of the absorbent article have an total open area of less than 1.4 mm² and typically not smaller than 0.15 mm². Apertures which are even smaller are usually not suitable for liquid transport at all and would only function as gas permeable apertures for example for breathability purposes. Preferably, the optional small apertures are in the range of 0.25 mm² to 0.4 mm².

The apertures are preferably substantially circular or polygonal. Their shape is limited by having a ratio of the largest to the smallest inner diagonal length in the range between 1 and 6, preferably 1 and 3. The total open area of all liquid transport apertures in the first layer is in the range of 10 % to 40 %, preferably 15 % to 35 % of the total area of the first layer. When approaching the upper limit of this total open area of the first layer, i.e. above a total open area of about 30 %, the distribution of apertures needs to be homogeneous, i.e. there should not be a specific area where more aperture areas are concentrated than elsewhere. First passage layers not having a homogeneous distribution of liquid passage ways are also contemplated by the present invention and would have a highest concentration of apertures in the area where liquid is expected to be discharged to.

The liquid transport apertures are formed in the film such that the walls of the apertures extend beyond the plane of the surface of the basic film, i.e. the film surface, before the film is apertured. The direction of these extending walls in the absorbent article is towards the garment facing surface of the article. The amount of extension of the walls of the apertures in the first layer is at least 0.3 mm beyond the film surface from which the walls of the apertures depend. Preferably the walls of the apertures form funnels or Ventury channels as is well-known in the art.

To ensure material stability the smallest distance between neighbouring apertures regardless of their particular shape and size is preferably at least 1.5 mm, preferably 2.0 mm. This distance is measured on the surface of the film on the side closest to the user facing surface of the absorbent article.

Also, as is typical for topsheets, the film material is preferably rendered hydrophilic to such a degree that the contact angle is less than 90° with distilled water upon first contact with the water. For films this can be achieved by surfactant treatment. For surfactant treated polymeric films providing the first layer it has been found that it is beneficial to use films where the surfactant is permanently fixed on the film surface. These are so called film materials with resin integrated surfactant. For these films even repeated wetting by distilled water would provide approximately the same contact angle as the first contact with distilled water.

In another preferred execution of the first layer of the topsheet the wearer facing surface is treated with an agent such that liquids are directed towards the apertures. Such agents can be silicone or teflon which provide the treated surface with a self-cleaning effect. This treatment can be in addition to the above-mentioned surfactant treatment.

Films such as those disclosed in EP-A-0 205 286, EP-A-0 165 208, EP-A-0 18 020, EP-A-0 59 506 or US-A-3,929,135 are explicitly referred to as suitable as first passage layer of the topsheet provided the requirements of the claims are met. Other suitable formed films, provided the requirements for the first passage layer are met, are described in U.S. Patent 4,324,246, U.S. Patent 4,342,314, U.S. Patent 4,463,045 and U.S. Patent 5,006,394. Particularly preferred microaperturing of formed film is disclosed in U.S. patent 4,609,518 and U.S. patent 4,629,643. These microapertures can also be included in the first passage layer of the topsheet provided they are less than 0.15 mm² and hence essentially provide breathability. Ways of making such films are well-known in the art and have also been disclosed in the above prior art references.

The second passage layer of the topsheet is provided by a second film material having apertures. Essentially this second passage layer can be identical to the first passage layer film except that large apertures should preferably be avoided. Due to the absence of hydrogel particles and if some large apertures are present they can be aligned with the large apertures in the first layer for not more than 20 % total open area, preferably not more than 10 % total area, otherwise the overlap could cause rewet and masking issues. Also the second film layer can have simple apertures which have no part of their walls depend from one surface of the film.

It is preferred that the second film material is at least as hydrophilic as the film material of the first layer in order to present no barrier for the liquid. Better yet it is more hydrophilic (or less hydrophobic) than the first film and creates a directing force for the liquid towards the absorbent structure after passing the first layer.

It is also important that both layers have about the same total open area. Preferably the second layer has 10 % larger, most preferably a more than 20 % larger total open area than the first film layer.

Since both layers of the topsheet, individually, are well-known in the art it is not necessary to elaborate on further details of the topsheet layers of the present invention but exemplify the performance of the here-to-forth unknown dual layer topsheet combination in the following examples:

### Absorbent article dimensions

The length, width and thickness of absorbent articles is typically defined by the desired use of such articles. However the present invention drastically improves masking such that even relatively thin articles can employ a large aperture film as the first topsheet layer. Hence the present invention specifically relates to absorbent articles having a maximum thickness of 8 mm or less, preferably 7 mm or less. This maximum thickness is determined as the caliper at the thickest part of the absorbent article. For articles with a profiled absorbent structure the thickest part is usually close to the center region of such an article. Caliper of this thickest part of the article can be measured by placing the article between two parallel plates applying a pressure of 0.0625 N/cm² and measuring the distance between the plates. The area for the pressure determination is the area defined by the circumference of the smaller of both plates which should extend approximately to an area of constant thickness of the absorbent article.

### Examples / Test procedure

The tests to analyse liquid intake rate, rewet and masking are simple tests to review the specific performance aspect of a product in relation to an alternative product. The results are intended to identify the qualitative difference between alternative executions and give an indication of the relative magnitude of improvements. Since primarily the topsheet combination distinguish the present invention from the prior art many backsheet and core combinations commercially available with the required characteristics will be available to evaluate alternative topsheet combinations.

For the tests described below an absorbent structure of two layers of thermally bonded wet layed fibers (having a composition of 45 % chemically stiffened, twisted and curled bulking fibers, 45 % cellulose fibers and 10 % bicomponent PP/PE fibers) provided the storage layer. This absorbent structure together with a polyethylene film backsheet was used as a base in combination with alternative topsheet combinations. The test absorbent article had a thickness of less than 2 mm.

The tested topsheet was made of a first film layer glued by a hotmelt adhesive to a second layer according to the present invention. The hotmelt adhesive was applied in a quantity of 3 g/m² by spiral gluing and the adhesive which was used was H2031, by Findley Euro B.V., from Netherlands.

The reference film layer was "DriWeave" film available from The Procter & Gamble Company, Cincinnati, Ohio, USA and commercialised on their Always (R) and Aldays (R) brands. This film is essentially identical with the first layer of the topsheet of the present invention but for the aperture size. Apertures in the reference film would only qualify as small apertures since they had 0.34 mm² open area. The total open area of the reference film was 31.28 % with 92 apertures per cm².

The first film layer according to the present invention had circular, slightly funnel shaped apertures of 1.7 mm² area for the large apertures and 0.20 mm² for the small apertures. The total open area was 32.82 % with 11.24 % due to the large apertures and 21.58 % due to the small apertures. The average number of apertures per cm² was 6.61 for the large apertures and 107.9 for the small apertures. The walls of the apertures including the basic film had a caliper of 0.427 mm while the film thickness was 0.0255 mm (such that the wall extended about 0.4 mm from the plane of the film).

The second layer according to the present invention was the above described "Dri Weave" film.

The reference second layer is available from Suominen, 29251 Nakkila, Finland, under reference F3200. It provides a caliper of 0.5 mm and basis weight of 50 g/m² at a void volume of 25 %.

The following test samples were prepared:

| Product Code: | A | B | C |
|---|---|---|---|
| first layer of topsheet | reference | large aperture film (according to present invention) | large aperture film (according to present invention) |
| second layer of topsheet | reference | reference | Dri Weave (according to present invention) |
| Core + Backsheet | - two layers of thermally bonded wet layed fibers plus backsheet of polyethylene - | | |

The tests were an acquisition test, rewet test and visual inspection for masking.

### Acquisition test procedure

This procedure measures a product's ability to "keep on absorbing" (acquisition decay) subject to a repeat assaults of fluid under a prescribed set of conditions. This procedure is recommended for multiple product comparisons.
This method evaluates the time required for the acquisition of given amounts of liquid during repeated imbitions (three in this case), at relatively high speed (about 3ml/sec) and under pressure of 1723.7 Pa (0.25 PSI), to model "in use" pressure while wearing.

Each product is layed down on a flat surface and an acquisition plate is placed on it. The acquisition plate comprises a rectangular plexiglass plate 70 x 220 x 8 mm with an aperture 22 mm in diameter formed therein. A cylinder 45 mm high and 22 mm in internal diameter is located over the aperture in sealing contact with the plate. The cylinder is filled with Artificial Menstrual Fluid (AMF), and a pressure of 1723.7 Pa (0.25 PSI) is applied to the plate, obtained with appropriate weights positioned on the plate, the pressure being that measured with reference to the portion of the product under the acquisition plate. The acquisition time is the time from the beginning of each imbition until the disappearance of the liquid from the interior of the cylinder. A waiting time of 10 minutes is left after each imbition before repeating the procedure.

### Rewetting test procedure

Drop 7 ml of Artificial Menstrual Fluid (AMF) over 90 secs onto the centre of a sanitary napkin.
Dropping area is 3x4 sqcm, with 4 cm being in the longitudinal direction of the sanitary napkin.
Let the pad stand for 20 min.
Place 7 layers of preweigthed blotting paper in the centre of the pad
Apply the pressure of 70 g/sqcm
Wait for 15 sec. with the pressure applied.
Remove the blotting paper and measure the weight
Final rewet result = Sum up the weight of liquid on 7 blotting papers.

| Composition of Artificial Method Fluid (AMF) used in the tests | |
|---|---|
| Defibrinated Sheep Blood (from UNIPATH) | = 50% |
| Phosphate Buffered Saline Solution(from SIGMA Chemie) | = 45% |
| Gastric Mucin type III(from SIGMA) | = 4% |
| Other ions (surfactant plus water) | =1% |

### Test results

The test results for acquisition and rewet can be found as index % in the following tables.
From visual inspection the masking of product A was better than the masking of product B and masking of the product according to the present invention, C, was better than either of the two alternatives A or B.

### Acquisition time results (index %)

### 1. Testfluid at viscosity of 7m Pas

| | | | |
|---|---|---|---|
| Cumulative amount of Liquid | 5 ml | 10 ml | 15 ml |
| Product A | 343 % | 305 % | 346 % |
| Product B | 271 % | 250 % | 271 % |
| Product C-basis | 100 % | 100 % | 100 % |

This result shows that the combination of the layers according to the present invention can provide the desired benefit of acquisition speed versus prior art combinations. In particular the improvement of C over B is surprising. While not wishing to be bound by theory it is believed that the additional liquid guiding and transporting between and below the two topsheet layers as well as the additional void volume provided by the dual layer topsheet provide this benefit.

### Rewet

In respect to rewet the problem prejudically expected with large aperture film topsheets is shown by the following rewet data. The topsheet according to the present invention addresses this problem and provides about the same rewet performance as the small aperture topsheet reference.

Rewet index of test fluid at viscosity of 11 m Pas

| | |
|---|---|
| Product A | 92 % |
| Product B | 200 % |
| Product C-basis | 100% |

## Claims

1. Absorbent article, having fast liquid intake, low rewet, and good masking performance, said article comprising a topsheet, a backsheet, and an absorbent structure placed between said topsheet and said backsheet, said article having a maximum thickness of 8 mm or less, said topsheet having a wearer facing surface and a garment facing surface and said topsheet comprising
- a first passage layer, said first passage layer providing said user facing surface of said topsheet, and
- a second passage layer, said second passage layer being placed between said first passage layer and said absorbent structure,
- both said passage layers being preferably joined to each other,
- said first passage layer being provided by a first film material having large apertures for liquid transport,
- said large apertures having an individual open area of 1.4 mm² or more, preferably in the range of 1.4 m² to 3.0 mm²,
- said large apertures having a total open area in the range from 5% to 20% of the total area of said first passage layer,
- said liquid transport apertures having a largest inner diagonal length and a smallest inner diagonal length, the ratio of said largest to said smallest inner diagonal length being in the range from 1 to 6,
- said liquid transport apertures having inner walls which depend at least 0.3 mm from the surface of said first film material, said inner walls depend in a direction towards said garment facing surface,
- said first film material being rendered hydrophilic such that it forms a contact angle of less than 90 degrees with distilled water upon first contact with distilled water,
- the second passage layer being provided by a second film material comprising small apertures for liquid transport,
- said small apertures in said second film material having an individual area in the range from 0.15 mm² to less than 1.4 mm²,
- said small apertures in said second film material having a total open area in the range from 10% to 40% of the total area of said second passage layer,
- said small apertures in said second film material having a largest inner diagonal length and a smallest inner diagonal length, the ratio of said largest to said smallest inner diagonal length being in the range from 1 to 3,
- said small apertures in said second film material preferably having inner walls which depend at least 0.3 mm from the surface of said second film, if such inner walls are present they preferably depend in a direction towards said absorbent structure,
- said second film material being preferably at least as hydrophilic as said first film material,
- said absorbent structure contains no hydrogel particles.

2. Absorbent article according to claim 1 wherein said second film material has only small apertures.

3. Absorbent article according to any of the preceding claims, wherein said large apertures have an individual open area in the range from 1.5 mm² to 2.5 mm².

4. Absorbent article according to any of the preceding claims wherein said large apertures have a total open area in the range from 10% to 20% of the total area of said first passage layer.

5. Absorbent article according to any of the preceding claims wherein said first passage layer is provided by a film material further having small apertures for liquid transport,
- said small apertures in said first passage layer have an individual open area in the range from 0.15 mm² to less than 1.4 mm²,
- the total open area of all said large and said small liquid transport apertures in said first passage layer is in the range from 10% to 40% of the total area of said first passage layer.

6. Absorbent article according to claim 5 wherein said small apertures in said first passage layer have an individual area in the range from 0.25 mm² to 0.4 mm².

7. Absorbent article according to claim 5 or 6 wherein the total open area of all said large and said small liquid transport apertures in said first passage layer is in the range from 15% to 35% of the total area of said first passage layer.

8. Absorbent article according to any of the preceding claims wherein the maximum thickness of said absorbent article is 7 mm or less.

9. Absorbent article according to any of the preceding claims wherein said absorbent structure consists only of said layer which is in direct contact with said topsheet.

10. Absorbent article according to any of the preceding claims wherein both said passage layers are joined directly to each other.

## Patentansprüche

1. Absorbierender Artikel, der eine schnelle Flüssigkeitsaufnahme, eine niedrige Wiederbefeuchtung und eine gute Maskierungsleistung aufweist, wobei der Artikel eine Oberschicht, eine Unterschicht und eine absorbierende Struktur, die zwischen der Oberschicht und der Unterschicht plaziert ist, aufweist, wobei der Artikel eine maximale Dicke von 8 mm oder weniger besitzt und die Oberschicht eine zum Träger hin weisende Oberfläche und eine zur Kleidung hin weisende Oberfläche aufweist, und die Oberschicht folgendes umfaßt:
- eine erste Durchgangsschicht, wobei die erste Durchgangsschicht die zum Benutzer weisende Oberfläche der Oberschicht liefert, und
- eine zweite Durchgangsschicht, wobei die zweite Durchgangsschicht zwischen der ersten Durchgangsschicht und der absorbierenden Struktur angeordnet ist;
- wobei diese beiden Durchgangsschichten vorzugsweise miteinander verbunden sind;
- wobei die erste Durchgangsschicht durch ein erstes Filmmaterial, das große Öffnungen für den Flüssigkeitstransport aufweist, geliefert wird;
- wobei die großen Öffnungen einzelnen Öffnungsgebiete von 1,4 mm² oder mehr, vorzugsweise im Bereich von 1,4 mm² bis 3,0 mm² aufweisen;
- wobei die großen Öffnungen insgesamt ein offenes Gebiet im Bereich von 5% bis 20% des gesamten Gebietes der ersten Durchgangsschicht aufweisen;
- wobei die Flüssigkeitstransportöffnungen eine größte innere diagonale Länge und eine kleinste innere diagonale Länge aufweisen, wobei das Verhältnis der größten inneren diagonalen Länge zur kleinsten inneren diagonalen Länge im Bereich von 1 bis 6 liegt;
- wobei die Flüssigkeitstransportöffnungen innere Wände haben, die mindestens 0,3 mm von der Oberfläche des ersten Filmmaterials abhängen, wobei die inneren Wände in einer Richtung auf die zur Kleidung zeigende Oberfläche abhängen;
- wobei das erste Filmmaterial hydrophil gehalten wird, so daß es einen Kontaktwinkel von weniger als 90 Grad mit destilliertem Wasser nach einem ersten Kontakt mit destilliertem Wasser bildet;
- wobei die zweite Durchgangsschicht durch ein zweites Filmmaterial geliefert wird, das kleine Öffnungen für den Flüssigkeitstransport umfaßt;
- wobei die kleinen Öffnungen in dem zweiten Filmmaterial ein einzelne Gebiete im Bereich von 0,15 mm² bis zu weniger als 1,4 mm² aufweisen;
- wobei die kleinen Öffnungen im zweiten Filmmaterial ein gesamtes offenes Gebiet im Bereich von 10% bis 40% des gesamten Gebietes der zweiten Durchgangsschicht aufweisen;
- wobei die kleinen Öffnungen im zweiten Filmmaterial eine größte innere diagonale Länge und eine kleinste innere diagonale Länge haben, wobei das Verhältnis der größten inneren diagonalen Länge zur kleinsten inneren diagonalen Länge im Bereich von 1 bis 3 liegt;
- wobei die kleinen Öffnungen im zweiten Filmmaterial vorzugsweise innere Wände haben, die mindestens 0,3 mm von der Oberfläche des zweiten Films abhängen, und wenn solche inneren Wände vorhanden sind, sie vorzugsweise in einer Richtung auf die absorbierende Struktur hin abhängen;
- wobei das zweite Filmmaterial vorzugsweise mindestens so hydrophil wie das erste Filmmaterial ist;
- wobei die absorbierende Struktur keine Hydrogelteilchen enthält.

2. Absorbierender Artikel nach Anspruch 1, wobei das zweite Filmmaterial nur kleine Öffnungen aufweist.

3. Absorbierender Artikel nach einem der vorhergehenden Ansprüche, wobei die großen Öffnungen einzelne Öffnungsgebiete im Bereich von 1,5 mm² bis 2,5 mm² aufweisen.

4. Absorbierender Artikel nach einem der vorhergehenden Ansprüche, wobei die großen Öffnungen ein gesamtes offenes Gebiet im Bereich von 10% bis 20% des gesamten Gebietes der ersten Durchgangsschicht aufweisen.

5. Absorbierender Artikel nach einem der vorhergehenden Ansprüche, wobei die erste Durchgangsschicht durch ein Filmmaterial geliefert wird, das weiter kleine Öffnungen für den Flüssigkeitstransport aufweist;
- wobei die kleinen Öffnungen in der ersten Durchgangsschicht einzelne Öffnungsgebiete im Bereich von 0,15 mm² bis zu weniger als 1,4 mm² aufweisen;
- wobei das gesamte offene Gebiet aller großen und der kleinen Flüssigkeitstransportöffnungen in der ersten Durchgangsschicht im Bereich von 10% bis 40% des gesamten Gebietes der ersten Durchgangsschicht liegt.

6. Absorbierender Artikel nach Anspruch 5, wobei die kleinen Öffnungen in der ersten Durchgangsschicht einzelne Gebiete im Bereich von 0,25 mm² bis 0,4 mm² aufweisen.

7. Absorbierender Artikel nach Anspruch 5 oder 6, wobei das gesamte offene Gebiet aller großen und der kleinen Flüssigkeitstransportöffnungen in der ersten Durchgangsschicht im Bereich von 15% bis 35% des gesamten Gebietes der ersten Durchgangsschicht liegt.

8. Absorbierender Artikel nach einem der vorhergehenden Ansprüche, wobei die maximale Dicke des absorbierenden Artikels 7 mm oder weniger beträgt.

9. Absorbierender Artikel nach einem der vorhergehenden Ansprüche, wobei die absorbierende Struktur nur aus der Schicht besteht, die sich in direktem Kontakt mit der Oberschicht befindet.

10. Absorbierender Artikel nach einem der vorhergehenden Ansprüche, wobei beide Durchgangsschichten direkt miteinander verbunden sind.

## Revendications

1. Article absorbant à admission de liquides rapide, faible remouillage, et bonne performance de masquage, ledit article comprenant une feuille de dessus, une feuille de fond et une structure absorbante placée entre ladite feuille de dessus et ladite feuille de fond, ledit article ayant une épaisseur maximale de 8 mm ou moins, ladite feuille de dessus ayant une surface faisant face à la personne qui la porte et une surface faisant face au vêtement, et ladite feuille de dessus comprenant :
- une première couche de passage, ladite première couche de passage fournissant ladite surface faisant face à l'utilisateur de ladite feuille de dessus, et
- une deuxième couche de passage, ladite deuxième couche de passage étant placée entre ladite première couche de passage et ladite structure absorbante,
- les deux dites couches de passage étant, de préférence, réunies l'une à l'autre,
- ladite première couche de passage étant réalisée avec un premier matériau pelliculaire présentant de grands orifices pour le transport de liquides,
- lesdits grands orifices ayant une surface ouverte individuelle de 1,4 mm², ou plus, comprise, de préférence, dans une plage allant de 1, 4 mm² à 3,0 mm².
- lesdits grands orifices ayant une surface ouverte totale comprise dans la plage allant de 5% à 20% de la surface totale de ladite première couche de passage,
- lesdits orifices de transport de liquides ayant une longueur diagonale intérieure supérieure et une longueur diagonale intérieure inférieure, le rapport entre lesdites longueurs diagonales intérieures supérieure et inférieure étant compris dans une plage allant de 1 à 6,
- lesdits orifices de transport de liquides présentant des parois intérieures qui pendent au moins de 0,3 mm de la surface dudit premier matériau pelliculaire, lesdites parois intérieures pendent dans une direction orientée vers ladite surface faisant face au vêtement,
- ledit premier matériau pelliculaire étant rendu hydrophile afin qu'il forme un angle de contact inférieur à 90 degrés avec de l'eau distillée lors d'un premier contact avec de l'eau distillée,
- la deuxième couche de passage étant réalisée avec un deuxième matériau pelliculaire comprenant de petits orifices pour le transport de liquides,
- lesdits petits orifices ménagés dans ledit deuxième matériau pelliculaire ayant une surface individuelle comprise dans une plage allant de 0,15 mm² à moins de 1,4 mm²,
- lesdits petits orifices ménagés dans ledit deuxième matériau pelliculaire ayant une surface ouverte totale comprise dans la plage allant de 10 % à 40 % de la surface totale de ladite deuxième couche de passage,
- lesdits petits orifices ménagés dans ledit deuxième matériau pelliculaire ayant une longueur diagonale intérieure supérieure et une longueur diagonale intérieure inférieure, le rapport entre lesdites longueurs diagonales intérieures supérieure et inférieure étant compris dans une plage allant de 1 à 3.
- lesdits petits orifices ménagés dans ledit deuxième matériau pelliculaire ayant, de préférence, des parois intérieures qui pendent au moins de 0,3 mm de la surface dudit deuxième film, si ces parois intérieures sont présentes, elles pendent, de préférence, dans une direction orientée vers ladite structure absorbante,
- ledit deuxième matériau pelliculaire étant, de préférence, au moins aussi hydrophile que ledit premier matériau pelliculaire,
- ladite structure absorbante ne contient pas de particules d'hydrogel.

2. Article absorbant selon la revendication 1, dans lequel ledit deuxième matériau pelliculaire ne présente que de petits orifices.

3. Article absorbant selon l'une quelconque des revendications précédentes, dans lequel lesdits grands orifices ont une surface ouverte individuelle comprise dans la plage allant de 1,5 mm² à 2,5mm².

4. Article absorbant selon l'une quelconque des revendications précédentes, dans lequel lesdits grands orifices ont une surface ouverte totale comprise dans la plage allant de 10% à 20% de la surface totale de ladite première couche de passage.

5. Article absorbant selon l'une quelconque des revendications précédentes, dans lequel ladite première couche de passage est réalisée avec un matériau pelliculaire présentant, en outre, de petits orifices pour le transport de liquides,
- lesdits petits orifices ménagés dans ladite première couche de passage ont une surface ouverte individuelle comprise dans la plage allant de 0,15 mm² à moins de 1,4 mm²,
- la surface ouverte totale de tous lesdits grands et petits orifices de transport de liquides ménagés dans ladite première couche de passage est comprise dans la plage allant de 10% à 40% de la surface totale de ladite première couche de passage.

6. Article absorbant selon la revendication 5, dans lequel lesdits petits orifices ménagés dans ladite première couche de passage ont une surface individuelle comprise dans la plage allant de 0,25 mm² à 0,4 mm².

7. Article absorbant selon la revendication 5 ou la revendication 6, dans lequel la surface ouverte totale de tous lesdits grands et petits orifices de transport de liquides ménagés dans ladite première couche de passage est comprise dans la plage allant de 15% à 35% de la surface totale de ladite première couche de passage.

8. Article absorbant selon l'une quelconque des revendications précédentes, dans lequel l'épaisseur maximale dudit article absorbant est de 7 mm ou moins.

9. Article absorbant selon l'une quelconque des revendications précédentes, dans lequel ladite structure absorbante est constituée seulement de ladite couche qui est en contact direct avec ladite feuille de dessus.

10. Article absorbant selon l'une quelconque des revendications précédentes, dans lequel lesdites couches de passage sont directement réunies l'une à l'autre.
